# EUROPEAN PATENT APPLICATION

(11) **EP 3 162 352 A1**
(43) Date of publication of application: **03.05.2017**
(21) Application number: 16195688.3
(22) Date of filing: 26.10.2016
(51) Int. Cl.: A61K 6/00, A61K 6/083

(54) **METHODS AND COMPOSITIONS OF REACTIVE MATERIALS FOR DENTAL PROCEDURES**

(30) Priority: 26.10.2015 US 201562246195 P; 10.06.2016 US 201662348235 P
(71) Applicant: Kerr Corporation, Orange, CA 92867 (US)
(72) Inventor: BOSISIO, Matteo R, Orange, CA 92867 (US); DRECHSLER, Ulf, Orange, CA 92867 (US); MERZLIKINE, Alexei G, Orange, CA 92867 (US); SOUNDARARAJAN, Gopi, Orange, CA 92867 (US); MEHRAB, Mohammad Reza, Orange, CA 92867 (US)
(74) Representative: Korenberg, Alexander Tal

(57) **Abstract**

Methods and systems for using a curable material for endodontic processes are provided. The method comprises using an electroactive composition to cure and optionally un-cure the material. Using an electroactive composition allows for easy introduction of the material without unwanted complexity or side effects. It also allows for easy removal and replacement if desired.

## Description

### CROSS-REFERENCE TO RELATED APPLICATIONS

This application claims priority from United States Provisional Patent Application Serial Number 62/246,195 filed on October 26, 2015, the contents of which are incorporated by reference in their entirety.

This application also claims priority from United States Provisional Patent Application Serial Number 62/348,235 filed on June 10, 2016, the contents of which are incorporated by reference in their entirety.

### FIELD OF TECHNOLOGY

This disclosure relates to dental restorative materials and uses thereof.

### BACKGROUND

Dental restorative materials include but are not limited to adhesives, cements, composites, impression materials, root canal obturation materials and/or core build-up materials. A number of difficulties manifest with the application of such materials. Some difficulties include properly positioning the material as well as incompatibility between conditions required to cure the material and other conditions required by the dental therapy protocol. Thus there is clear utility in novel compositions, improved dental materials, and methods of their application.

The art described in this section is not intended to constitute an admission that any patent, publication or other information referred to herein is "prior art" with respect to this invention, unless specifically designated as such. In addition, this section should not be construed to mean that a search has been made or that no other pertinent information as defined in 37 CFR § 1.56(a) exists.

### BREIF SUMMARY OF THE INVENTION

To satisfy the long-felt but unsolved needs identified above, at least one embodiment of the invention is directed towards a method and a composition for applying a dental treatment. The composition may be deposited within a mouth. The composition may comprise a polymer and an electroactive composition. The composition may be hardened or cured upon exposure to an electric field, electromagnetic radiation, light emission, and any combination thereof.

In at least one embodiment, the curing is accomplished through low-voltage activation. The composition may comprise co-dissolved carbene precursors grafted onto polyamidoamine units. This composition may be activated at a low-voltage (for example 2 Volts or more or less) facilitating tunable crosslinking within the composition's matrix and/or on electrode surfaces. The crosslinking increases intermolecular bonding, hardening the composition. When the applied voltage is discontinued, the crosslinking terminates. Thus, crosslinking initiation and propagation may be voltage and time dependent, enabling tuning of both material properties and the composition's cured strength.

The method may further comprise de-curing the composition upon exposure to an electric field, electromagnetic radiation, light emission, and any combination thereof. Optionally at least a portion of the de-cured composition may be removed.

The composition may further comprise diazirine, a diazirine derivative and/or poly-diazirine.

The composition may be placed within a tooth, tooth cavity, within a root canal, may seal a prosthetic dental appliance to a hard oral surface or to oral tissue, and/or may be pressed against a portion of the mouth to form an impression of that portion of the mouth.

The polymer may be one item selected from the group consisting of: a synthetic polymer, a self-adhering polymer, a radiopacity polymer, a sonic-reactive polymer, and any combination thereof.

The composition may further comprise one item selected from the group consisting of: camphorquinone curable material, gutta percha, trans-poly isoprene material, rubber, thermoplastic, gold, silver, titanium, magnesium silicate, glass-crystalline ceramic particles (including but not limited to silicates, zirconium oxide, lithium disilicates,and aluminum oxide), latex, zinc oxide, barium sulfate, mineral wax, bioceramic material, hydroxyapatite (HAP) substitutes, MTA, and any combination thereof. The method may further comprise the step of positioning the composition using a non-contact motive force. The non-contact motive force may be selected from the group consisting of: iontophoresis, electro-osmosis, electrophoresis, electrolysis, electroporation, iontophoresis, and any combination thereof.

The composition may adhere to both a first portion of the mouth and a second different portion of the mouth.

### DETAILED DESCRIPTION OF THE INVENTION

As used herein, **"Biocompatible"** generally refers to having the ability to be in contact with tissue, bone, teeth, or other portions of a living system without producing any adverse effect, it includes but is not limited bio-inert materials.

As used herein, **"Electrocuring"** generally refers to the process of hardening (making more viscous) a material upon exposure of the material to an electrical current, it may be the result of chemical (including but not limited to steric) changes occurring to carbene groups in the material caused by the current, it may be accomplished through the use of at least one: anode, cathode, or both.

As used herein, "RC" generally refers to root canal.

As used herein, **"Polydispersity"** generally refers to the size distribution of dispersed materials (for example polymers), it is expressed in terms of the weight average molecular weight of the material divided by the number average molecular weight of the material.

At least one embodiment may be a reactive material useful in a dental restorative procedure. The material may be chemically reactive to an emission and/or may be directed towards a location by an emission. Representative emissions include electric current, light, UV light, IR light, sonic waves, thermal heat, radiation, and any combination thereof.

The materials can be appreciated when considering deficiencies innate to many prior art materials. In operation, many of the dental restorative materials rely on available technologies for instant chemical curing. For example, instant, or substantially instant, chemical curing may be triggered by either temperature, actinic radiation, chemical or moisture cure or a combination of more than one of these methods.

One drawback associated with prior art materials is "shrinkage". During the curing process, prior art materials shrink or reduce their volume. In contrast, in at least one embodiment the innovative material undergoes little to no shrinkage or undergoes slight expansion. For example when cured the volume of the material changes by between -0.1 to 10%, preferably 0.1 to 1%.

Other drawbacks associated with conventional chemical curing may relate to increased-temperature conditions necessary for curing. In certain instances increased-temperature conditions required for chemical curing may overheat the affected tissue. In some instances, increased-temperature conditions may affect other heat-sensitive material.

Under certain circumstances, increased-temperature conditions required for curing may conflict with the limited ranges of mechanical properties of the restorative materials.

Increased-temperature conditions required for curing may also require sometimes difficult to administer temperature control of setting during application and handling. Such control may, at times, require a system and/or method for manipulating the temperature during the curing process. As a result of certain environmental, or other, conditions, poor or no adhesion may be obtained through chemical curing that relies on increased-temperature conditions.

In addition, some drawbacks associated with conventional chemical curing may relate to light conditions necessary for curing using actinic radiation such as visible, IR, and UV light. For example, the efficiency of the radiation cure can be limited by the ability of the light to penetrate the material.

Other issues that may affect conventional chemical curing that relies on light conditions may include the risks of high dermal tissue sensitivity and overheating.

In addition, some drawbacks associated with conventional chemical curing may relate to complex delivery requirements. Some cured materials require coordinating the delivery of two or more different materials requiring complicated feeding equipment. Other drawbacks may relate to mixing requirements that involve maintaining homogeneity of certain materials prior to the mixing phase. Some drawbacks may also relate to difficulties associated with handling and application of chemicals having relatively small viscosity ranges, limited setting time ranges once mixed, and limited working time ranges once mixed.

Also, some drawbacks associated with the conventional chemical curing may relate to moisture conditions involved with, and/or required for, certain types of chemical curing.

Such moist conditions may limit the field of application of such curing. Such moisture conditions may affect the mechanical properties of such materials as well as adhesion associated with the materials.

Many, if not all, of the above-mentioned curing strategies are also strongly limited by being characterized as typically irreversible reactions (except, under certain conditions, thermoset reactions), typically moisture-dependent reactions and typically substrate-dependent reactions. Characteristics of such substrates, which may under certain conditions limit an application of such strategies, may include, but not be limited to, hydrophilicity, acidity of surfaces (metals), and types of ceramic substrate. Some, if not all, of the above-mentioned curing strategies are also limited as the strategies may be associated with fixed adhesive strength with limited ranges, fixed viscoelastic properties with limited ranges, fixed cohesive strength with limited ranges and shrinkage issues during curing which may cause residual stress or create voids.

Further, many, if not all, of the above-mentioned curing strategies depend heavily on whether the specific material used is brittle and hard (highly-crosslinked) with low loss modulus (low viscoelastic properties) or not. Such material characteristics may affect long-term durability due to limited plasticity.

In view of the foregoing, it would be desirable to provide systems and methods for curing strategies that overcome the above-described drawbacks.

In contrast, as the following details, the inventive materials facilitate a number of useful attributes.

The materials are capable of working and setting time of material needed to correctly place and shape it in-situ, whether that material is adhesive, composite, cement, impression material and/or root canal obturation material.

The materials control and/or limit the level of cure (and depth of cure in the photo-curable material), especially in composite material.

The materials control the level of moisture in the oral cavity to preferably match each specific dental composition requirement, such requirements which may include hydrophilicity, hydrophobicity and moisture sensitivity.

The materials may facilitate the removal of a cemented crown during a crown re-do procedure or inspection of dental implants, the latter being, at times, a required procedure.

The materials are effective for the removal of a cemented orthodontic appliance as well as for the removal of previously-placed composite material, root canal obturation material, sealants or other dental material.

The materials correctly adhere an indirect restoration (inlay, onlay, overlay, crown and/or bridge) in view of placement restrictions, working time to place the handwork restrictions, and unforeseen, and often uncontrollable, environmental factors.

The materials match, and/or approximates, an advantageous, and/or optimum, viscosity that facilitates the placing of the dental compositions (preferably to obtain correct flowability of material) and facilitates the final mechanical properties -- e.g., viscoelastic moduli, hardness, toughness, and/or strength -- of the finally-cured dental composition.

The materials effect sanitization of dental restorative composition and elimination of excess dental restorative composition in any direct or indirect procedures, including root canal obturation procedures. This is especially useful as it is well known that sanitization of, and removal of excess cement during, final crown cementation is critical and impacts the longevity of the restoration (bacteria sticking to cement excess).

In at least one embodiment, a material useful in direct and/or indirect dental restorations or oral tissue fixation comprises one or more electrochemically-hardenable compounds. The material may be biocompatible and/or may have configurable viscoelastic, mechanical and adhesive properties.

A representative example of such an electrochemically-hardenable compound includes but is not limited to Voltaglue (from Nanyang Technological University, Singapore). Also representative examples and methods of their use are described in one, some, or all of Patent Publication WO 2015/108487 A1, and scientific papers: Novel On-Demand Bioadhesion to Soft Tissue in Wet Environments, by V. Mogal et al., Macromol. Biosci., issue 14, pp. 478-484 (2014) (hereinafter "Mogal"), and Adhesive curing through low-voltage activation, by J. Ping et al., Nature Communications, Article number: 8050 (2015), all of whose contents are hereby incorporated by reference in their entirety.

In at least one embodiment, the material may undergo a quick-cure (which occurs within time duration of between 1-1000 seconds) which is electrochemically-activated. The material may comprise free-radical precursors that may be triggered via reductive or oxidative electrochemistry. The composition may be used for dental restoration and/or tissue fixation.

Depending on the target application, the electroactive dental composition may comprise a biocompatible polymer, a biopolymer and/or a polymer with covalently attached free-radical precursors, and/or particles with covalently attached free-radical precursors. Such compositions may initiate crosslinking under application of low voltages. Such a crosslinking mechanism is an example of "electrocuring".

Depending on the specific target application, the polymer and/or biopolymer and/or particles may be functionalized with electroactive free-radical precursors. Electroactive dental compositions may be formulated for a number of uses such as: 1) an adhesive or cement dental material, 2) a composite or a filling material for dental cavities, fissures, or lesions, 3) an impression material to accurately record three dimensional anatomy of the oral hard and soft tissues, 4) an obturation material used in the root canal, 5) a material for core build-up or any other suitable dental application that utilizes adhesion, and 6) sealing and/or filling substrates. An exemplary list of such materials suitable for use in such compositions may include but is not limited to: solvents, surfactants, stabilizers, fillers and/or other additives selected and weighted according to the application.

Such compositions may be electrically conductive and/or may contain additional conductive particles. Such compositions and/or particles may include diazirines and/or poly-diazirines, e.g. particles functionalized with carbene precursors (preferably diazirine) and/or polymers functionalized with diazirine groups, that can by synthesized via organic chemistry methods known to a person skilled in the art.

In such compositions, the free-radical precursor may be triggered via reductive electrochemistry. In some embodiments, the crosslinking of the polymer backbone can be controlled by switching "ON" the functional groups as the voltage rises above a certain threshold. When the voltage falls below the threshold, the crosslinking process may stop.

Under the certain circumstances, the crosslinking may occur simultaneously, or substantially simultaneously, in the bulk of the electrocurable materials of the polymer, or other suitable material, and at the interfaces between said material and the dental or oral surfaces that are being worked upon. The crosslinking may preferably not be limited by the depth as opposed to current UV-curing mechanism which are depth-limited.

In some embodiments, the user may control, on-demand and preferably in real-time, the following parameters: a) the setting time of the polymer, which would preferably enable virtually-unlimited working time in order to be able to place the material and rapidly set the material in response to a preferably pre-determined trigger; b) the material's viscoelastic and final mechanical properties which may dictate that the material can be flowable at time of application and be hardened at subsequent stages to ease placement; c) depending on the procedure, may enable the user to select, in real time, the elastic properties (storage modulus (G')) and the viscous properties (loss modulus (G'')) with the help of analog or electronic feedback for precise generation of G'/G"; d) the strength of the adhesive bond, including but not limited to material mechanical properties and substrate adhesion, which can preferably be selected real time via analog or electronic feedback; and e) the cure or polymerization which, in certain embodiments, may preferably not be depth-dependent.

The dental hardenable composition according to certain embodiments may preferably be set in moist and/or wet environments and allow optimal wetting of all substrates.

Electrocuring initiation may preferably be triggered once adherents are joined, properly positioned and wetted by the composition. In certain embodiments, the dental composition may be designed to generate a radical oxidizer within the eventual moisture-enabling setting in moisture environments.

The crosslinking may preferably be driven by free radicals produced at the surfaces of the substrates, the bulk of the substrates, organic-inorganic interfaces, the surface and/or bulk of inorganic fillers, the surface and/or bulk of reinforcement particles, the surface or bulk of the polymer(s), and any combination thereof. Some or all of the polyers, substrates, and/or fillers may be coated with materials comprising carbene precursors. In certain embodiments sacrificial conducting electrode(s) are used and the patient tissue interfaces represent the low conductive counter electrode. Such a system preferably substantially operates independent of depth. As used herein, the term "depth", refers to the distance of a location from the surface of the substrate.

The dental hardenable composition according to some embodiments may be reversibly converted in response to certain triggers to a soft material in order to be removed. The suitable triggers are either chemical, electrical, sonic, magnetic, optical, thermal or a combination thereof. In certain embodiments the chemical approach requires the addition of a chemical group that undergoes bond breakage on command via application of thermal energy, electrical current or chemical exposure to strong acids or bases. Electrochemical reversibility can be obtained via application of continuous or alternating voltages at different voltage levels or a predetermined sequence thereof. In yet another embodiment electrochemical reversibility can be achieved with the addition of chemical groups that are photo reactive, as for example azo compounds.

The electroactive dental materials described below may be used in conjunction with other biocompatible materials or apparatuses to dispense, apply the material, apply or conduct electrical current, cure and de-cure the electroactive dental material, and/or control the level of cure and provide feedback of cure, depending on the dental procedure.

Electroactive compositions may contain a biocompatible polymer or biopolymer with covalently attached electroactive groups. Electroactive compositions may contain inorganic fillers/particles with covalently attached electroactive groups. Such electroactive groups include but are not limited to carbene groups and/or precursors thereof, and which can be triggered via oxidative or reductive electrochemistry. Other free-radical precursors may be for example from the vinyl or azo groups. Within the azo groups, diazonium salts are highly versatile. Some examples of suitable diazonium salts are aryl-diazo compounds, derivatives of diazonium. Other suitable salts may include arylsulfonium, diaryliodonium salts, derivates of arylsulfonium salts, or combinations. The electroactive groups may be used to functionalize the biocompatible dental materials. Dental materials used in dental products may be functionalized on the molecular scale and/or on the supramolecular scale. On the molecular level, diazirine functional groups can be added to monomers, oligomers, and fillers, using the methods from synthetic organic chemistry known to a person skilled in the art. Macroscopic and microscopic objects on the supramolecular scale can be functionalized by grafting diazirine moieties to their surface, for example, as described in Mogal, by first grafting the surface with amino groups, and then functionalizing them with N-hydroxysuccinimide-functionalized diazirine.

In at least one embodiment, the electrocurable composition starts to harden once the applied voltage surpasses a certain threshold. This may be but is not limited to: plus or minus 0.1-10 Volts (or more or less), preferably plus or minus 1-5 Volts. While certain voltage ranges have been set forth herein, it should be noted that any sufficient voltage suitable to enable the redox chemistry may preferably be used. Viscosity may be time dependent and the hardening build up can be designed according to, for example, the polymer structure, the functionalized groups, their concentration and/or the additives of the compositions (filler, plasticizers, etc.). Time dependent viscosity of the electroactive dental composition may allow the material to be placed in low viscosity and selectively hardened by the user during placement if needed or completely quickly hardened in response to a predetermined trigger. The electrocuring can be fast (full cure in less than 20 seconds or preferably 5 or fewer seconds) and preferably be substantially independent depth dependencies. In some embodiments, a thickness of 10mm (or more or less) can be cured/de-cured. In at least one embodiment the thickness which is de-cured is only a portion of the overall thickness. In at least one embodiment the emission (such as but not limited to light) which causes the de-curing has a wavelength of between 50-1500 nm (or more or less) preferably 350-400 nm.

Several methods of applying the electrical current are described according to the embodiments:

The electric potential may be applied via a single electrode and the body serves as a ground.

The single electrode may be a conductive tool of any form, a coating of conductive material, a root canal ("RC") carrier obturator, an impression tray with electrodes or coating, and any combination thereof.

The dental composition may cure and/or adhere against non-conductive organic or inorganic substrates.

The dental composition bulk may cure / de-cure under application of voltages via a constant, punctual, or variable voltage source (with or absent the addition of conductive particles/nanoparticles).

The conductive or partially conductive electrode may be a portion of, engaged to, and/or integrated within a dental prosthethic device (such as an indirect restoration or orthodontic ("ortho") bracket) driving voltages applied to the device.

The implant abutment may be a conductive or partially conductive electrode. Driving voltages may be applied to the abutment.

The electrostatic potential may be applied within one, two, or more electrodes.

The electrodes may be thin (less than, equal to, or more than 1 mm) and/or extremely close each other (less than, equal to, or more than 1 mm) to create small voltages changes thus triggering local electrocure or de-cure/softening of the material (if electrically reversible).

The electrodes may be placed across tissue (e.g. two sides of a crown, two sides of a root, across gum sides, etc.)

The dental composition may cure and/or adhere against non-conductive organic or inorganic substrates.

The dental prosthetic device (such as an indirect restoration or ortho bracket) is the conductive or partially conductive electrode and driving voltages may be applied to the abutment.

The implant abutment or tooth structure (prep tooth or remaining tooth) may be the conductive or partially conductive electrode and driving voltages may be applied to the prosthethic device or tooth structure (such as a prep tooth or remaining tooth).

In at least one embodiment, an adhesive layer is formed via diazirine-driven crosslinking. This may be engineered in such a way as to be degraded on demand for debonding purposes. There are many ways to achieve this; for example, by incorporating photocleavable functional groups into the polymer backbone, such as diazo groups (1), that can break the chains upon exposure to UV light. In structure (1), R1-R4 can be selected to optimize the absorption spectrum of the compound, and to control the stability of the carbon-centered radicals formed after degradation, and R5 and R6 are the linkers connecting this moiety to the polymer backbone.

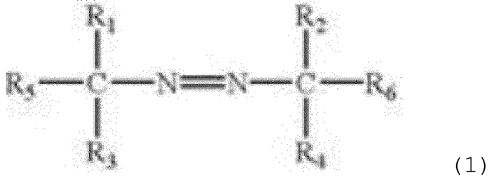

In one embodiment light-induced molecular motion due to photo-isomerization of azobenzene groups or aryl azo compounds may trigger material breakdown. Another option to degrade the adhesive layers is to incorporate hydrolyzable groups, such as ester groups, and trigger the degradation by pH changes upon exposure of the adhesive to acids or bases. The latter can also be achieved by incorporating photoacid or photobase generators into the material.

Yet another option is to use thermally-degradable groups such as peroxy- or tertiary- ester moieties and/or pericyclic reactive groups.

U.S. Patent Application No. 14/077,957 and U.S. Patent Application No. 11/275,246 describe thermally-labile groups that can be used in combination with the present invention. Each of these patent applications is incorporated by reference herein in their respective entireties.

In some particular embodiments the polymer, or biopolymer may be a dental material, i.e. it is known in the art to be useful in dental care (for example it may be FDA approved). Without specific limitations, described herein are four sets of materials suitable for dental applications: 1) dental resins (adhesive/cements/composites/sealers), 2) polymers used in root canal obturation, 3) polymers used as impression material for the accurate replica of oral hard and soft tissue (impression materials); and/or 4) FDA-approved polymers suitable for oral tissue fixation.

Dental resins comprise monomers characterized by high hydrolysis stability and are particularly suitable as diluting monomers because of their relatively low viscosity. Preferred polyfunctional (meth)acrylic acid derivatives with high hydrolysis stability are cross-linking pyrrolidones such as 1,6-bis(3-vinyl-2-pyrrolidonyl)-hexane, bisacrylamides such as methylene or ethylene bisacrylamide and bis(meth)acrylamides such as N,N'-diethyl-1 ,3-bis(acrylamide)-propane, 1 ,3-bis(methacrylamido)-propane, 1 ,4-bis (acrylamido)-butane or 1 ,4-bis(acryloyl)-piperazine which can be synthesized by reacting the corresponding diamines with (meth)acrylic acid chloride.

Such resins may be used in dual cure (two-component system) or light cure (typically visible range, wavelength between about 380 and about 800 nm (or more or less) or, in some embodiments, wavelength in the UV or IR range), or combination, in order to perform direct or indirect restorations, e.g., composite to fill cavities, seal root canals or cement orthodontic or prosthetic appliances. Such polymers are well-suited for the electroactive compositions for indirect and direct procedures, e.g. filling cavities, seal root canals or cementing prosthetic appliances. A pre-wetting coating may be applicable to the substrates to be bound in order to optimize the electrical conductivity and/or the adhesion.

In other embodiments, an electroactive hardenable resin that has the ability to be softened/uncured on demand is of particular interest for root canal filling.

Some limitations in conventional resin-based technology for the obturation of root canal that may be solved by the invention include: a) Possible obturation material shrinkage issue; b) Moisture dependency; c) Limited light penetration/diffusion in RC interstices; and d) Circumstances where RC retreatment is inapplicable.

Example of applications according to the embodiments may include: a) electrocurable composite; b) electroactive adhesive or cement; and/or c) electroactive root canal sealer/filler.

Depending on the bioadhesive shear and bond strength of the final electroactive dental composition undercuts may be required or created to optimize the retention of the dental direct or indirect restoration. In some embodiments (prosthetic) undercuts may optimize the retention of the prosthetic appliance when the electroactive dental composition is at its hardened states, and facilitate the removal of the dental appliance when the electroactive dental composition is switched to flowable state.

Thermoplastic polymer may also be used for root canal ("RC") obturation. It may be used in the presence of or absence of natural or synthetic gutta percha, an isoprene, latex, and any combination thereof (in any one, some, or all of the alpha, beta, or amorphous state). Chemically, gutta-percha is a polyterpene, a polymer of isoprene, or polyisoprene, specifically (trans-1,4-polyisoprene). The cis structure of polyisoprene is the common latex elastomer. While latex rubbers are amorphous in molecular structure, gutta-percha (the trans structure) crystallizes, leading to a more rigid material.

Electroactive functionalization described above may be used for electroactive cure and un-cure.

Other optional components in this embodiment are: ZnO, Bismuth oxide or other radiopaque filler, antimicrobial, and/or bacteriostatic compounds.

Examples of embodiments may include the following:
1) Root canal sealant and filling comprising the polymer with cross-linkable groups that are triggered by electrochemical methods. The crosslinkable groups may preferably utilize diazirine, or other suitable chemistry. The crosslinking could be triggered by electrodes or light or other emission sources inserted into the filler material or by electrodes augmented by the conductive particles in the material. Such embodiments may utilize radiopacity to enhance placement.
2) Root canal sealant and obturator systems in which the obturator is electrically conducting and the sealant utilizing electrochemically triggered crosslinking such as using diazirine-based chemistry. The electric potential is applied via the obturator (which can be a gutta-percha obturator with the embedded conductive particles). The obturator may include two or more surface areas electrically isolated from each other serving as (+) and (-) electrodes.
3) Injection via a conductive cannula in the RC and subsequent electrocuring.
4) The cannula may be left in the RC.
5) During retreatment the cannula which was left in the RC may serve to apply electrical current.

An additional benefit of the electroactive dental composition used for RC filling is disinfection. Thus, a method for disinfecting root canals by utilizing diazirine chemistry is also provided. Because electrochemical conversion of diazirine produces highly reactive carbene, this method will not only crosslink the polymers (sealant) but also may react with the bacteria present in the root canal pores. To increase the efficacy of this step, a low viscosity diazirine-derivative containing washing solution can be injected into the root canal (and electrolyzed).

The electrocuring aspect of this invention enables the filling of dental cavities, including root canals, or the cementation of prosthetics with several advantages: being a single component cure system with on-demand adhesion (unlimited working time, quick setting time); adhesion to preferably all substrates and reduced or no moisture sensitivity; variable viscosity; and variable mechanical strength / viscoelastic properties / adhesion (user defined).

The materials may also be used to produce impression materials. One or more of the following polymer(s) may be functionalized pursuant to the strategy as set forth above to create an electroactive hardenable material that could melt or otherwise dissipate in response to a pre-determined trigger.
1) Polyether, based on cross-linking of polyether chains via cationic polymerization of aziridine rings using an aromatic sulfonate ester as an initiator;
2) C-type Silicones, based on cross-linking polycondensation reaction of hydroxyl-terminated polysiloxane prepolymer with tetra alkoxy silanes catalyzed by dibutyl-tin dilaurate (DBTD);
3) A-type Silicones, based on cross-linking polyaddition reaction of vinyl terminated polysiloxane polymer with methylhydrogen silicone cross-linking agent in the presence of platinum catalyst.

Also, hybrid solutions have become available recently. Vinylsiloxanether combines characteristics of polyether and those of A-silicones and therefore provides the flowability in combination with hydrophilicity and elastic properties.

The electroactive impression material can undergo substantially on-demand cure. The dental composition may allow clinicians substantially unlimited time to correctly place the material in the patient's mouth and to select the required level of viscosity before and during application via application of electrical voltages in short pulses. The system may allow for feedback of viscosity. Once ready, the electrical applied voltage will finally harden the material to take the actual impression. In one embodiment it is preferable that the material is not reversible or undergoes irreversible cure/setting (avoid re usage for cross contamination within patients, stability of the replica over time, etc.). In yet another embodiment, the tray typically filled chair side is a pre-filled tray with electroactive composition.

The materials may also be used for tissue fixation. Oral surgery often requires surgical suture of soft tissues. An embodiment of the invention is resorbable tissue glue for oral soft tissues. A polymer for use according to the invention is set forth below:

The main polymeric strand or backbone can be any bioresorbable polymer. In at least one embodiment it is one or more items selected from the group consisting of: polyethylene glycol (PEG), PEG fatty acid esters, poly-L-lactic acid (PLLA), poly(lactide- co-glycolide) (PLGA), poly caprolactone (PCL), polyvinyl pyrrolidone (PVP), polyvinyl alcohol (PVA), collagen, chitosan, hydroxy propyl cellulose, polyamides, polyglycerol esters of fatty acids, polysaccharides, polyesters, other bioresorbable polymers, and any combination thereof. The polysaccharide may be selected from the group consisting of dextran, chitosan, heparin, hyaluronic acid, alginates, starch, glycogen, amylose, amylopectin, cellulose, xylan, and numerous other natural and synthetic polysaccharides, and any combination thereof.

In at least one embodiment the bioresorbable polymer is in the form of a thin film matrix. It can be blended for independent tailoring of thin film mechanical properties to match soft tissue, controlled drug release, conductivity, and any combination thereof.

The dental compositions described in this text may be filled with appropriate fillers or other additives to deliver the desired properties.

Depending on the final application, the dental composition may further include one of or more of the following components: plasticizer, x-ray visible particles, remineralization additives, antimicrobial additives or other additives.

The following known materials, or similar materials, may be used, in the appropriate proportions, to fill the non-diazirine portions of compositions.

Adhesives: may comprise some or all of: acetone, 2-hydroxyethyl methacrylate ethanol, and 2-hydroxy-1,3-propanediyl bismethacrylate, Representative examples of adhesives include: Optibond, Optibond SoloPlus, OptiBond All in One by Kerr corporation, Orange, CA.

Cements: may comprise some or all of: 1,6-hexanediyl bismethacrylate, 2-hydroxy-1,3-propanediyl bismethacrylate, 7,7,9(or 7,9,9)-trimethyl-4,13-dioxo-3,14-dioxa- 5,12-diazahexadecane-1,16-diyl bismethacrylate, 3-trimethoxysilylpropyl methacrylate, and 1,1,3,3-tetramethylbutyl hydroperoxide. Representative examples of cements include: Maxcem by Kerr Corporation, Orange, CA.

Composites: may comprise some or all of: glass, oxide, Silicon dioxide (1-methylethylidene)bis(4,1-phenyleneoxy-2,1-ethanediyloxy-2,1-ethanediyl) bismethacrylate (1-methylethylidene)bis[4,1-phenyleneoxy(2-hydroxy-3,1-propanediyl)] bismethacrylate 2,2'-ethylenedioxydiethyl dimethacrylate, Poly(oxy-1,2-ethanediyl), α,α'-[(1-methylethylidene)di-4,1-phenylene]bis[ω-[(2-methyl-1-oxo-2-propen-1-yl)oxy]-2,2'-ethylenedioxydiethyl dimethacrylate, 3-trimethoxysilylpropyl methacrylate, (1-methylethylidene)bis[4,1-phenyleneoxy (2-hydroxy-3,1-propanediyl)] bismethacrylate, 2-hydroxyethyl methacrylate, 2-hydroxyethyl methacrylate bismethacrylate, 2-hydroxy-1,3-propanediyl bismethacrylate, Poly(oxy-1,2-ethanediyl), and α,α'-[(1-methylethylidene)di-4,1-phenylene]bis[ω-[(2-methyl-1-oxo-2-propen-1-yl)oxy]. Representative examples of composites include: SonicFill 2, Herculite Ultra, and Vertise Flow by Kerr Corporation, Orange, CA.

Impression Materials: may comprise one or more of: Platinum, 1,3-diethenyl-1,1,3,3-tetramethyldisiloxane complexes, Quartz, Siloxanes, Silicones, branched nonylphenol, and ethoxylated nonylphenol. Representative examples of impression materials include: Take 1 Advanced and AlgiNot by Kerr Corporation, Orange, CA.

Root canal obturation materials may include: gutta percha, sealers, mineral trioxide aggregate (MTA), and bioceramics.

Core build-up materials may include some or all of: Poly(oxy-1,2-ethanediyl), α,α'-[(1-methylethylidene)di-4,1-phenylene]bis[ω-[(2-methyl-1-oxo-2-propen-1-yl)oxy]- 1 2,2'-ethylenedioxydiethyl dimethacrylate, 7,7,9(or 7,9,9)-trimethyl-4,13-dioxo-3,14-dioxa- 5,12-diazahexadecane-1,16-diyl bismethacrylate, alkali fluorosilicates(Na), and 3-trimethoxysilylpropyl methacrylate. A representative example of a core build up material is CoreRestore 2 by Kerr Corporation, Orange, CA.

The apparatus and methods of using the compositions described herein are illustrative. It is to be understood that other embodiments may be utilized and that structural, functional and procedural modifications may be made without departing from the scope and spirit of the present disclosure.

The steps of the methods may be performed in an order other than the order shown and/or described herein. Embodiments may omit steps shown and/or described in connection with the illustrative methods. Embodiments may include steps that are neither shown nor described in connection with the illustrative methods.

Illustrative method steps may be combined. For example, one illustrative method may include steps shown in connection with another illustrative method.

Some apparatus may omit features shown and/or described in connection with illustrative apparatus. Embodiments may include features that are neither shown nor described in connection with the illustrative methods. Features of illustrative apparatus may be combined. For example, one illustrative embodiment may include features shown in connection with another illustrative embodiment.

### ILLUSTRATIVE EMBODIMENTS

1) A method for filling a cavity in a tooth using a compound, said compound comprising a synthetic polymer and an electroactive composition, said method comprising:
   Placing the compound in a cavity; and
   curing the compound using an electric field.
1A) The method of claim 1 further comprising uncuring the compound using an electric field.
1B) The method of claim 1 wherein the composition comprises diazirine, a diazirine derivative and/or poly-diazirine.
2) A method for sealing a root canal using a compound, said compound comprising a synthetic polymer and an electroactive composition, said method comprising:
   placing the compound in a root canal; and
   curing the compound using an electric field.
2A) The method of claim 2 further comprising uncuring the compound using an electric field.
2B) The method of claim 2 wherein the composition comprises diazirine, a diazirine derivative and/or poly-diazirine.
3) A method for cementing prosthetic dental appliances using a compound, said compound comprising a synthetic polymer and an electroactive composition, said method comprising:
   placing the compound on oral hard and/or soft tissue; and
   curing the compound using an electric field.
3A) The method of claim 3 wherein the composition comprises diazirine, a diazirine derivative and/or poly-diazirine.
4) A composition for filling a cavity in a tooth, said composition comprising:
   a compound, said compound comprising:
      a synthetic polymer and
      an electroactive composition, wherein the electroactive composition comprises diazirine, a diazirine derivative and/or poly-diazirine.
4A) To the extent that the composition for filling a cavity set forth in 4) includes any one or more of camphor quinone curable components or gutta percha, then the inventive composition contemplates removing some or all of the camphor quinone curable components or gutta percha or fiber core in favor of a replacement including, at least partially, diazirine or a diazirine composition.
5) A method for obturating a root canal in a tooth using a compound, said compound comprising a polymer and an electroactive composition, said method comprising
   placing the compound within the root canal; and
   curing the compound using an electric field.
5A) The method of claim 5 further comprising uncuring the compound using an electric field.
5B) The method of claim 5 wherein the composition comprises diazirine, a diazirine derivative and/or poly-diazirine.
6) A composition for obturating a root canal in a tooth, said composition comprising:
   a compound, said compound comprising:
      Gutta Percha or other similar suitable compounds; and
      an electroactive composition comprising diazirine,
      a diazirine derivative and/or poly-diazirine.
6A) To the extent that any of the composition for obturating set forth in 6) includes any one or more of camphor quinone curable components or gutta percha, then the inventive composition contemplates removing some or all of the camphor quinone curable components or gutta in favor of a replacement including, at least partially, diazirine or diazirine composition.
7) A method for adhering impression material for the accurate replica of oral hard and/or soft tissue, said method using a compound, said compound comprising a polymer and an electroactive composition, said method comprising:
   placing the compound on the oral hard and/or soft tissue in order to obtain an impression of the oral hard and/or soft tissue; and
   curing the compound using an electric field.
7A) The method of claim 7 further comprising uncuring the compound using an electric field.
7B) The method of claim 7 wherein the composition comprises diazirine, a diazirine derivative and/or poly-diazirine.
8) A composition for use as dental impression material, said composition comprising:
   a compound, said compound comprising:
      a polymer; and
      an electroactive composition, said electroactive composition including diazirine, a diazirine derivative and/or poly-diazirine.
8A) To the extent that any of the composition for use as dental impression material, as set forth in 8), includes any one or more of camphor quinone curable components or gutta percha, then the inventive composition contemplates removing some or all of the camphor quinone curable components or gutta percha in favor of a replacement including, at least partially, diazirine or diazirine composition.
9) A method for adhering a first oral tissue to a second oral tissue, said method using a compound, said compound comprising a polymer and an electroactive composition, said method comprising:
   placing the compound on the first tissue and the second tissue in order to adhere the first tissue to the second tissue; and
   curing the compound using an electric field.
9A) The method of claim 9 further comprising de-curing the compound using an electric field.
9B) The method of claim 9 wherein the composition comprises diazirine, a diazirine derivative and/or poly-diazirine.
9C) The method of claim 9 wherein at least one of the tissues is restorative and/or biological.
10) A composition for adhering a first oral tissue to a second oral tissue, said composition comprising:
   a polymer, said polymer comprising OptiBond XTR TM, OptiBond SoloPlus TM, OptiBond All in One TM or other similar suitable compounds; and
   an electroactive composition, wherein the electroactive composition comprises diazirine, a diazirine derivative and/or poly-diazirine.
10B) The method of claim 10 wherein at least one of the tissues is restorative and/or biological.
11) To the extent that any of the composition for adhering a first oral tissue to a second oral tissue set forth in 10) includes any one or more of camphor quinone curable components or gutta percha, then the inventive composition contemplates removing some or all of the camphor quinone curable components or gutta in favor of a replacement including, at least partially, diazirine or diazirine composition.
12) A composition, the composition comprising a polymer, a reactive composition, and an oral component, wherein the reactive composition is curable in response to exposure to light, electric charge, or both, the oral component is one item selected from the group consisting of a tooth, a tooth cavity, a jaw bone, saliva, tooth enamel, mouth tissue, tongue tissue, inner cheek tissue, pulp, dentine, gum, root canal, root tip, any portion thereof, and any combination thereof.

At least one embodiment is directed towards irrigation, cleaning and filling of a root canal. The root canal in a tooth is typically a long, curved canal. In a healthy tooth, the pulp of the tooth is located within the canal. When the pulp becomes deeply infected, the pulp can no longer remain within the canal and must be removed. Typically, removal of infected pulp, and removal of other unwanted debris in the tooth canal, is accomplished using mechanical means.

Following removal of the pulp, the canal is filled with a biologically inert substance. Examples of such filling materials include, but are not limited to, gutta-percha, sealers (cement), mineral trioxide aggregate (MTA) and bioceramics.

Conventionally, gutta-percha and/or another biologically-inert substance, is inserted into the root canal using hand-actuated devices. In at least one embodiment the material is one or more of the appropriate materials described in patent documents US 20130337414 A1 and WO 2015/006087 A1, the contents of which are incorporated by reference in their entirety.

Disinfection with current methods is limited by many factors, including the availability of chlorine concentration after storage. Disinfection with current methods is also limited because of the difficulty of disrupting biofilm and pulp residuals in order to reach microbial colonies present in the infected root canal system.

Thus, it is desirable to remove the pulp and/or other unwanted debris (such as proteins and/or lipids) from the root canal using a contactless transport mechanism. It is further desirable to move gutta-percha or other biologically-inert substance into the root canal using a contactless transport mechanism.

In at least one embodiment, the material is used to improve root canal disinfection and/or to remove the pulp and/or other unwanted debris from the root canal using a contactless transport mechanism. This contactless transport mechanism may facilitate the positioning of gutta-percha, another biologically-inert substance, an electrochemically-hardenable compound, and any combination thereof into a root canal or other tooth location, using a contactless transport mechanism. In at least one embodiment, by altering the flow-rate and/or flow direction of the non-contact transport mechanism, precise material placement may be achieved. This allows for the treatment of very small or hard to reach locations, cavities, or infections that might otherwise be difficult to reach.

In at least one embodiment, the non-contact transport mechanism includes but is not limited to: iontophoresis, electro-osmosis, electrophoresis, electrolysis, electroporation and/or sonophoresis. All of these mechanisms are non-invasive methods of propelling matter of a certain drug or chemical material or charged substance using a motive force sufficient to drive such material. In certain embodiments, electrolysis can preferably be implemented in situ directly in the root canal complex to drive non-spontaneous chemical reaction at the site of interest (root canal/pulp) - e.g., to create slightly acidic electrolyzed water - in situ.

In certain embodiments, high voltage short pulses may be used to generate free radicals creation in sodium hypochlorite solution, enhancing an antimicrobial effect. For the purpose of illustration, the electrolysis process described herein is typically how salt water chorine generators work. In such processes, the DC current for iontophoresis may be used to generate chlorine by itself given a salt solution placed in the root canal.

In some embodiments, the infected pulp and/or other unwanted debris may be disinfected and removed from the root canal using iontophoresis. Certain embodiments may involve inserting water, electrolyzed water, sodium hypochlorite - e.g., bleach -, acid or other flushing material (collectively, hereinafter "flushing material") into the root canal. Certain embodiments may involve flushing with lactam compounds. Lactam compounds may be used to disrupt biofilm. These may be effective in parts per billion concentrations.

The flushing material may be delivered into the root canal and extracted from the root canal (along with any infected root tissue or unwanted debris that was perturbed by the initial propelling of water) using a dental irrigation apparatus. Such an apparatus may utilize positive irrigation and/or negative irrigation. A representative device is the Endovac, manufactured by Kavo Kerr of Orange, California. The flushing material may be further propelled into the root canal interstices and extracted from the root canal (along with any infected root tissue or unwanted debris that was perturbed by the initial propelling of water) using electromotive force sufficient to drive such ionic flushing material (iontophoresis).

Microbubbles or microspheres (particles) may be dispersed into the flushing material. Drugs, disinfectants, tissue disruptors, such as enzymes (e.g. pepsin, trypsin, papain), mineralization agents (e.g. hydroxyapatite (HAP) substitutes, fluorides, metal fluorides nanoparticles, enzymes) or others (active agents), may be encapsulated in, or incorporated into, microbubbles or microspheres. Particles are preferably charged, and thus sensitive to electrical or magnetic fields. In addition, uncharged particles may be moved via electro-osmosis.

During application of electrical field, the particles may be driven to the suitable target. In addition, particles may be modified by having a ligand. Ligands bind to biomaterials such as tissue, protein, and enzymes, causing a change on conformation which effects a noticeable change in the biomaterial. Ligands include but are not limited to substrates, inhibitors, activators, and neurotransmitters. Such ligands may be coupled via a coupling agent. The ligand may preferably be able to specifically bind to target tissue or may happen in response to triggering of specific triggers - e.g., higher intensity field application of chemo-mechanical triggers, sonic, ultrasonic, thermal or other source of energy, releasing active agent locally.

Collection of particles may be mono-dispersed or poly-dispersed depending on the application. Particles are usually smaller than one millimeter in diameter, and sub-micrometer particles may be used depending on the application. Preferably the size of particles ranges from 1 to 100 micrometer size. It may have a polydispersity between 1-500, or more or less.

In one embodiment of the application, the particles may be bubbles filled with gas, e.g. air, perfluorocarbon or other gas used in medical application. Microbubbles can oscillate when sonic energy is applied and may resonate, generating shear and bulk waves in the flushing material thereby enhancing debridement and tissue disruption. The internal pressure of the engineered microbubble may be slightly higher than the external pressure. In response to specific triggers, such as described above, the bubbles may break generating additional shock waves at the site of interest. Such shock waves increase the tissue debridement and biofilm disruption. Microbubble shell and core may comprise material usually used in ultrasound contrast imaging.

The flushing material may interact with the electrical charge and enhance its disinfectant properties.

An iontophoresis device may include an active electrode and a counter electrode coupled to opposite poles of a voltage source. The source can deliver constant current and/or constant voltage (DC) or alternating current (and/or voltage AC) depending on the application. The apparatus comprises one ground electrode section (inactive electrode) that contributes to the maintenance of the required potential difference between the biological tissue and the iontophoresis electrode section (active electrode). The inactive electrode can be placed in contact with the patient's body or directly in contact with a portion of the tooth to be treated. The active electrode can be placed at the foramen of the root canal complex or directly inserted into the root canals.

In one embodiment of the invention the active electrode is one or more microneedles. The microneedle may be alternatively used to deliver or to evacuate flushing material during irrigation. Multiple needles may be used simultaneously. The needle may be affixed to a part of the irrigation device described above. The microneedle may deliver sonic or ultrasonic vibration, thermal changes or other sources of energy.

In certain embodiments, in order to avoid contact within the root canal walls, the active electrode(s) may be protected via insulating spacers (rings). Multiple spacers can be applied to the needles forming, for example, a protective network. Alternatively, the needle can be partially over-coated with a protective substance. In yet another alternative, no spacers or over-coating substance may be used, and the needle may just be placed in the canal.

After the canal is sufficiently prepared, cleaned and disinfected, iontophoresis, or other suitable method, may then be used for enhanced remineralizing of dentinal walls. Such remineralizing may be for the purpose, for example, of sealing dentinal tubules from the inner part of the tooth.

Non-contact motive forces such as iontophoresis or induced magnetic fields can be additionally used for the controlled introduction into the root canal mineralization particles or filling material, such as gutta-percha (including alpha, beta, and/or amorphous configuration), trans-poly isoprene materials, rubber, thermoplastics, gold, silver, titanium, magnesium silicate, glass-crystalline ceramic particles (e.g. silicates, zirconium oxide, lithium disilicates, aluminium oxide), latex, zinc oxide, barium sulfate, mineral wax, bioceramic material, hydroxyapatite (HAP) substitutes, MTA or other filling material, either biologically inert or bioactive material, that are sensitive to electrical charge or magnetic field or other suitable method.

In certain embodiments, the filling material, preferably bioceramic, gutta-percha or other biologically inert material may be conditioned for use in iontophoresis by the introduction of an electrical or magnetic charge to the filling material. Electrical or magnetic charge may be introduced to the filling material, charged nano- or micro-particles or other biologically inert material by adding charged ions to the filling material. The charged ions are responsive to the applied electrical or electromagnetic field. Such a charge may preferably allow the filling material or other biologically active or inert material to be introduced in a controlled fashion into the root canal using iontophoresis or magnetic field application.

In some embodiments, filling material may be introduced in a controlled fashion into the root canal using micro-bubbles. Micro-bubbles may contain a substance and, when the micro-bubbles are transported to a desired location within the root canal, may be ruptured using an applied current or voltage, or other source of energy, thereby depositing the substance contained within the micro-bubbles to the desired location.

Such micro-bubbles may preferably contain gutta-percha or other biological material, which may be manufactured and delivered in the form of micro- or nano- particle itself. Such particles may, in certain embodiments, contain charge. Accordingly, filling material may be iontophoretically transported into the root canal targets using charged particles, which, upon arrival of the micro-bubbles at a desired location within the root canal targets may be electronically ruptured to release, in a targeted fashion, the filling material.

Some exemplary embodiments of non-contact migrations of dental materials include:
1. A method for disinfecting and removing infected tissue and debris from a root canal, the method comprising:
   applying iontophoresis, an electrical field or a magnetic field to propel a fluid into the root canal;
   perturbing the infected tissue and debris within the canal using iontophoresis; and
   flushing the fluid and the perturbed tissue and debris from the root canal using iontophoresis, an electrical field or a magnetic field.
2. A method for disinfecting and removing infected tissue and debris from a root canal, the method comprising:
   generating controlled electrolysis in situ.
3. A method for disinfecting and removing infected tissue and debris from a root canal, the method comprising:
   generating controlled electrophoresis in situ using, said generating using 3-dimensional imaging to control energy level of electrophoresis.
4. A method for inducing enhanced remineralizing of dentinal walls for sealing dentinal tubules from the inner part of the tooth into a root canal, the method comprising:
   applying bioactive mineralizing material; and
   applying electrical charge to transfer the mineralizing agent to the target biological tissue.
5. A method for introducing filling material, such as hydroxyapatite ("HAP") particles, metal fluoride nanoparticles, mineral trioxide aggregate ("MTA"), bioceramic or other biologically inert material, into a root canal, the method comprising:
   applying iontophoresis, an electrical field or a magnetic field to propel gutta-percha or other biologically inert material into the root canal; and
   terminating application of the iontophoresis to control the positioning of the filling material within the root canal.
6. A composition of matter comprising:
   filling material, such as hydroxyapatite ("HAP") particles, metal fluoride nanoparticles, mineral trioxide aggregate ("MTA"), bioceramic or other biologically inert material, said filling material comprising charged ions and/or particles that are responsive to iontophoresis or magnetic field.
7. A composition of matter comprising:
   A bioactive mineralizing material, said material comprising charged ions and/or particles that are responsive to iontophoresis or magnetic field.
8. A composition of matter comprising:
   micro- or nano- particles to deliver active agent at a target site with a root canal upon a remote trigger.
9. A composition of matter comprising:
   micro-bubbles, said micro-bubbles comprising a shell and core, where the core is gas filled, the core having an internal pressure higher than the external pressure, the shell being responsive to acoustic, iontophoresis or other source of energy triggers to create shock waves, shear wave or bulk waves in a target region of the root canal.
10. Apparatus to deliver iontophoresis, dispense and release irrigation and filling compositions in a targeted fashion.
11. Apparatus to enhance mineralization and root canal filling by the means of iontophoresis.
12. A method of sealing a root canal using iontophoresis, an electrical field or a magnetic field.

Exemplary embodiments may include the following methods:
1. A method for applying a dental treatment, the method comprising depositing within a mouth a composition, said composition comprising a polymer and an electroactive or light reactive composition and curing the composition using a respective electric field, light emission, or combination thereof.
2. The method of claim 1 further comprising de-curing the composition using an electric field or light emission and optionally removing at least a portion of the de-cured composition.
3. The method of claim 1 wherein the composition comprises diazirine, a diazirine derivative and/or poly-diazirine.
4. The method of claim 1 in which the composition is placed within a cavity within a tooth.
5. The method of claim 1 in which the composition is placed within a root canal.
6. The method of claim 1 in which the composition remineralizes a root canal.
7. The method of claim 1 in which the composition seals a root canal.
8. The method of claim 1 in which the composition seals a prosthetic or orthodontic dental appliance to a hard oral surface or to oral tissue.
9. The method of claim 1 in which the polymer is one item selected from the group consisting of: a synthetic polymer, a self-adhering polymer, a radiopacity polymer, a sonic-reactive polymer, and any combination thereof.
10. The method of claim 1 in which the composition further comprises one item selected from the group consisting of: camphorquinone curable material, gutta percha, trans-poly isoprene material, rubber, thermoplastic, gold, silver, titanium, magnesium silicate, glass-crystalline ceramic particles (e.g. silicates, zirconium oxide, lithium disilicates, aluminium oxide), latex, zinc oxide, barium sulfate, mineral wax, bioceramic material, hydroxyapatite (HAP) substitutes, MTA, and any combination thereof.
11. The method of claim 1 further comprising the step of positioning the composition using a non-contact motive force.
12. The method of claim 11 in which the non-contact motive force is selected form the group consisting of:
   iontophoresis, electro-osmosis, electrophoresis, electrolysis, , sonophoresis, and any combination thereof.
13. The method of claim 1 in which the composition is pressed against a portion of the mouth and forms an impression of that portion of the mouth.
14. The method of claim 1 in which the composition adheres to both a first portion of the mouth and a second different portion of the mouth.

While this invention may be embodied in many different forms, there are described in detail herein specific preferred embodiments of the invention. The present disclosure is an exemplification of the principles of the invention and is not intended to limit the invention to the particular embodiments illustrated. All patents, patent applications, scientific papers, and any other referenced materials mentioned herein are incorporated by reference in their entirety. Furthermore, the invention encompasses any possible combination of some or all of the various embodiments described herein and/or incorporated herein. In addition the invention encompasses any possible combination that also specifically excludes any one or some of the various embodiments described herein and/or incorporated herein.

The above disclosure is intended to be illustrative and not exhaustive. This description will suggest many variations and alternatives to one of ordinary skill in this art. All these alternatives and variations are intended to be included within the scope of the claims where the term "comprising" means "including, but not limited to". Those familiar with the art may recognize other equivalents to the specific embodiments described herein which equivalents are also intended to be encompassed by the claims.

All ranges and parameters disclosed herein are understood to encompass any and all subranges subsumed therein, and every number between the endpoints. For example, a stated range of "1 to 10" should be considered to include any and all subranges between (and inclusive of) the minimum value of 1 and the maximum value of 10; that is, all subranges beginning with a minimum value of 1 or more, (e.g. 1 to 6.1), and ending with a maximum value of 10 or less, (e.g. 2.3 to 9.4, 3 to 8, 4 to 7), and finally to each number 1, 2, 3, 4, 5, 6, 7, 8, 9, and 10 contained within the range. All percentages, ratios and proportions herein are by weight unless otherwise specified.

This completes the description of the preferred and alternate embodiments of the invention. Those skilled in the art may recognize other equivalents to the specific embodiment described herein which equivalents are intended to be encompassed by the claims attached hereto.

## Claims

1. A composition for use in dental treatment, the composition comprising a polymer and an electroactive or light reactive composition, wherein the composition is curable using a respective electric field, light emission, or combination thereof.

2. The composition of claim 1 comprising an oral component, wherein the oral component is one item selected from the group consisting of a tooth, a tooth cavity, a jaw bone, saliva, tooth enamel, mouth tissue, tongue tissue, inner cheek tissue, pulp, dentine, gum, root canal, root tip, any portion thereof, and any combination thereof.

3. The composition of claim 1, wherein the composition is de-curable using an electric field or light emission, and the dental treatment comprises optionally removing at least a portion of the de-cured composition.

4. The composition of any preceding claim, wherein the composition comprises diazirine, a diazirine derivative and/or poly-diazirine.

5. The composition of any preceding claim, wherein the dental treatment comprises placing the composition within a cavity within a tooth.

6. The composition of any preceding claim, wherein the dental treatment comprises placing the composition within a root canal.

7. The composition of any preceding claim, wherein the dental treatment comprises remineralizing a root canal using the composition.

8. The composition of any preceding claim, wherein the dental treatment comprises sealing a root canal using the composition.

9. The composition of any preceding claim, wherein the dental treatment comprises sealing a prosthetic or orthodontic dental appliance to a hard oral surface or to oral tissue using the composition.

10. The composition of any preceding claim, wherein the polymer is one item selected from the group consisting of: a synthetic polymer, a self-adhering polymer, a radiopacity polymer, a sonic-reactive polymer, and any combination thereof.

11. The composition of any preceding claim, comprising one item selected from the group consisting of:
camphorquinone curable material, gutta percha, trans-poly isoprene material, rubber, thermoplastic, gold, silver, titanium, magnesium silicate, glass-crystalline ceramic particles (e.g. silicates, zirconium oxide, lithium disilicates, aluminium oxide), latex, zinc oxide, barium sulfate, mineral wax, bioceramic material, hydroxyapatite (HAP) substitutes, MTA, and any combination thereof.

12. The composition of any preceding claim, wherein the dental treatment comprises positioning the composition using a non-contact motive force.

13. The composition of claim 12, wherein the non-contact motive force is selected form the group consisting of: iontophoresis, electro-osmosis, electrophoresis, electrolysis, sonophoresis, and any combination thereof.

14. The composition of any preceding claim, wherein the dental treatment comprises pressing the composition against a portion of the mouth and forming an impression of that portion of the mouth.

15. The composition of any preceding claim, wherein the composition is adhereable to both a first portion of the mouth and a second different portion of the mouth.
